# EUROPÄISCHE PATENTSCHRIFT

(11) **EP 0 810 001 B1**
(45) Veröffentlichungstag und Bekanntmachung des Hinweises auf die Patenterteilung: **08.10.2008**
(21) Anmeldenummer: 97108525.3
(22) Anmeldetag: 27.05.1997
(51) Int. Cl.: A61M 25/00

(54) **Vorrichtung zur Behandlung von Harnblasen-Entleerungsstörungen des Mannes und der Frau**
Device for treating male and female bladder emptying disorders
Dispositif de traitement des troubles pour vider la vessie chez l'homme et la femme

(30) Priorität: 28.05.1996 DE 19621420
(43) Veröffentlichungstag der Anmeldung: 03.12.1997
(73) Patentinhaber: Engel, Konrad Dr.med., D-83674 Gaissach (DE)
(72) Erfinder: Engel, Konrad, Dr., 83674 Gaissach (DE); Engel, Kilian, 83674 Gaissach (DE)
(74) Vertreter: Nätebusch, Roderich

(56) Entgegenhaltungen:
- DE-A- 3 743 139
- US-A- 3 331 371
- US-A- 4 932 938
- US-A- 5 360 403

## Beschreibung

Die Erfindung betrifft eine Vorrichtung zur Behandlung von Harnblasenentleerungsstörungen des Mannes und der Frau gemäß dem Oberbegriff des Anspruches 1.

Unter Harninkontinenz versteht man den unwillkürlichen Verlust von Urin aus der Harnblase und Harnröhre. Die Ursachen sind entweder eine direkte Schädigung des Verschlußapparates (Schließmuskel) der Harnblase, meist als Folge einer Prostataoperation oder durch Infiltration eines Prostatakarzinoms beim Mann oder einer Schließmuskelverletzung durch Geburten bei der Frau. Weitere Ursachen einer Harninkontinenz sind Nervenschädigungen als Folge von Stoffwechselkrankheiten, wie z.B. Diabetes mellitus oder als Folge von Traumen der die Harnblase und deren Verschlußmechanismus versorgenden Nerven, wie Schlaganfall, Tumoroperationen im Beckenbereich oder Rückenmarksverletzungen.

Bei der Inkontinenz als Folge einer Nervenschädigung kann meist der Verschlußmechanismus der Blase nicht mehr ausreichend geöffnet werden, sodaß der Urinverlust (Inkontinenz) erst nach maximaler Füllung der Harnblase in die Harnblase nicht ganz entleerender Menge und unkontrollierbar eintritt. Die Folgen sind neben dem Einnässen eine Überdehnung der Harnblase und in vielen Fällen der Rückstau des Urins bis in beide Nieren mit folgender Nierenschädigung. Von den verschiedenen Formen der Inkontinenz sind meist Menschen höheren Lebensalters betroffen.

Zur Behandlung und Behebung der Harninkontinenz sind je nach Ursache derselben und je nach Geschlecht bereits vielfältige Methoden bekannt, die jedoch in schwerwiegenden Fällen meist nicht wirksam genug sind oder einer Operation mit oder ohne Implantat bedürfen und nicht in jedem Fall frei von Nachteilen sind.

Bei der Inkontinenz durch teilweisen oder völligen Funktionsverlust des Verschlußapparates der Harnblase ist vor allem beim Mann die Anwendung eines operativ implantierbaren künstlichen Blasenschließmuskels nach "Scott" bekannt (AMS 800 der Firma American Medical Systems). Das Implantat ist sehr teuer und soll nur von erfahrenen Ärzten implantiert werden. Schwerwiegende Infektionen des das Implantat umgebenden Gewebes oder Nekrosen desselben durch Druck mit notwendiger Entfernung des Implantats wurden wiederholt beobachtet. Bei Frauen ist bei dieser Form der Inkontinenz eine physikalische Therapie oder eine wenig belastende Operation ohne Implantat häufig erfolgreich.

Eine weitere bekannte Vorrichtung zur Behebung der Inkontinenz des Mannes besteht aus einer sogenannten Penisklemme oder einem Penoring, wobei durch Druck von außen auf den Penis bzw. die Harnröhre ein mehr oder minder traumatisierender Druck ausgeübt wird. Außerdem besteht die Gefahr des Verrutschens in der Unterwäsche mit Einnässen.

Weiterhin sind für beide Geschlechter außen am Körper getragene Urinauffangsysteme mit Urinbeutel (Urinale) oder absorbierende Hilfsmittel (Einwegwindeln) bekannt, die zum Auffangen des Urins dienen und Hautreizungen durch den Urin, Geruchsbelästigung und damit soziale Isolation zur Folge haben.

Außerdem ist eine Vorrichtung für die Inkontinenz der Frau bekannt, wobei die Harnblase über einen kurzen aus der Harnröhre ragenden Katheter durch manuelle Betätigung eines im Scheidenvorhof vorhandenen Ventils entleert wird (EP 0 407 218 A1). Das im Scheidenvorhof liegende Ventil dürfte zu einer Besiedelung der Vorrichtung durch Scheidenvorhofbakterien führen.

Seit neuestem ist eine Vorrichtung für die Inkontinenz der Frau unter dem Namen "Reliance TM" auf dem Markt (US-A-5.090.424), die aus einem aufblasbaren Harnröhreneinsatz besteht, der mehrmals täglich vor jeder Blasenentleerung ähnlich einem Tampon völlig entfernt und anschließend durch eine neue Vorrichtung ersetzt werden muss, was sehr aufwendig ist.

Es ist auch schon ein Inkontinenzkatheter mit einem durch die Harnröhre in das Blasenlumen eines Menschen einführbaren lang gestreckten Rohrteil bekannt (US-A-3 331 371), welches in dem Blasenlumen durch einen das Rohrteil umgebenden aufblasbaren Halteballon gehalten wird und welches an seinem in das Blasenlumen eingeführten Endabschnitt ein Ventil aufweist. Dieses Ventil kann in unterschiedlicher Weise realisiert sein.

Bei einer Ausführungsform weist das Rohrteil an seinem in das Blasenlumen eingeführten Endabschnitt ein im unbetätigten Ventilzustand durch eine bewegbare Gummikugel verschlossenes Fluideintrittsloch auf, welches mit einem längs des betreffenden Rohrteiles verlaufenden Durchgang verbunden ist. Die Gummikugel ist mit einer Seilanordnung verbunden, deren eines Ende aus der Harnröhre herausgeführt sein muss, damit durch Ziehen an diesem Seilanordnungsende die Gummikugel aus ihrer Dichtungsanlage an dem Fluideintrittsloch herausführbar ist, so dass dann Harnflüssigkeit aus dem Blasenlumen durch das Rohrteil abgeführt werden kann. Dadurch treten bei dieser Realisierung des bekannten Inkontinenzkatheters jedoch entsprechende Probleme durch eine von außen in die Blase aufsteigende Besiedelung mit Bakterien auf, wie dies im Zusammenhang mit einer anderen bekannten Vorrichtung oben erwähnt worden ist.

Bei einer anderen Ausführungsform weist das Rohrteil an seinem in das Blasenlumen eingeführten Endabschnitt ein im unbetätigten Ventilzustand offenes Fluideintrittsloch auf, welches mit einem längs des betreffenden Rohrteiles verlaufenden Durchgang verbunden ist und welches durch einen mittels eines Gases oder eines Fluids aufblasbaren Dichtungsballon verschließbar ist. Wie und durch welche Einrichtung das Aufblasen des betreffenden Dichtungsballons erfolgt, ist in dem betreffenden Zusammenhang allerdings nicht angegeben.

Das Oberbegriff des Anspruchs 1 bezieht sich auf diese Ausführungsform.

Schließlich sind Vorrichtungen für die Inkontinenz des Mannes bekannt, mit denen grundsätzlich eine Reihe der vorstehend aufgezählten Nachteile vermieden werden kann (US-A-4.946.449, DE-OS 40 14 369 = US-A-4.932.938, EP-A-0 265 207, EP 0 543 309 B1). Diese bekannten Vorrichtungen bestehen im wesentlichen aus einem in die männliche Harnröhre einführbaren Katheter, der an seinem proximalen Endabschnitt einen mit einem Fluid (z.B. Wasser) füllbaren und dadurch erweiterbaren Balllon aufweist. Dieser Ballon dichtet die Harnblase am Eintritt in die Harnröhre ab und sichert den Katheter gegen ein ungewolltes Herausgleiten. Distal von dem Ballon ist ein mit Fluid füllbarer zweiter Ballon angeordnet, der im eingesetzten Zustand des Katheters außerhalb des Blasenschließmuskels in der Harnröhre liegt und auf diese Weise ein ungewolltes Verschieben des Katheters.in das Blaseninnere verhindert. Die Länge des Katheters ist derart bemessen, dass sein distales Ende im eingesetzten Zustand vollständig im Penis aufgenommen ist, und in dem distalen Endabschnitt ist ein Ventil angeordnet, das von außerhalb durch die Wandung der Harnröhre hindurch im Penisbereich tastbar ist. Das Ventil ist beispielsweise ein Schnabel-, Lippen-, Kugel- oder Schiebeventil, dessen normalerweise geschlossener Zustand sich durch Druck zwischen zwei Fingern in den geöffneten Zustand überführen läßt, sodaß die Miktion stattfinden kann. Diese am distalen Endabschnitt des Katheters angeordneten bekannten Ventile haben mehrere gravierende Nachteile. Der Lumenquerschnitt eines Katheters beträgt höchstens 5-6 mm, weshalb die Herstellung von auf diese Dimensionen miniaturisierter Lippen- oder Schnabelventile schwierig oder kostengünstig überhaupt nicht möglich ist. Auf dem geschlossenen Ventil lastet in Öffnungsrichtung durch den Blaseninnendruck ein Flüssigkeitsdruck von bis zu 100 cm Wassersäule, sodaß die Gefahr des Umschlagens des Ventils nach außen und damit ein Funktionsverlust vorprogrammiert ist. Das für lange liegende Katheter fast ausschließlich verwendete Silikonmaterial hat eine nur geringe gummielastische Rückstellkraft, sodaß Lippen- und Schnabelventile aus diesem Material einer metallelastischen Federvorrichtung bedürfen, um eine Rückkehr des geöffneten Ventils in eine abdichtende, geschlossene Position zu erreichen. Diese Federvorrichtung in den erwähnten geringen Dimensionen zu verwirklichen ist ebenfalls technisch sehr aufwendig. Metallische Vorrichtungen in der Katheterwand führen außerdem zu einer Verhärtung derselben und erhöhen die Gefahr von druckbedingten Schädigungen der empfindlichen Harnröhrenschleimhaut. Alle genannten am distalen Katheterende angebrachten Ventile führen zu einem deutlichen Verlust an Katheterlumen. Dies gilt vor allem für Kugel- oder Schiebeventile, sodaß bei diesen in geöffnetem Zustand kein ausreichender Harnfluß gewährleistet ist. Nicht verformbare Ventilelemente, wie z.B. Kugeln, neigen außerdem zu vermehrter Verkrustung durch die im Urin gelösten Stoffe.

Der Erfindung liegt daher die Aufgabe zugrunde, eine Vorrichtung zur Behandlung und Behebung insbesondere der männlichen Harninkontinenz der eingangs genannten Art so auszubilden, daß das Katheterventil leicht herstellbar ist, durch eine ausreichende elastische Rückstellkraft sicher schließt, auch bei hohen Blaseninnendrücken sich nicht selbstständig öffnet, das Katheterlumen weitgehend in seinem ganzen Querschnitt zur willkürlichen Drainage des Harns aus der Blase zur Verfügung steht und das Einführen der Vorrichtung in die Harnröhre und deren Entfernung daraus einfach ist. Außerdem sollte die Vorrichtung nach dem gleichen Grundprinzip, aber nach Anpassung an die anders gearteten anatomischen Verhältnisse der Frau, auch zur Behandlung und Behebung der Harninkontinenz der Frau geeignet sein.

Erfindungsgemäß wird mit einer Vorrichtung der eingangs genannten Art diese Aufgabe gelöst durch die Ausgestaltung der Vorrichtung gemäß dem Kennzeichen des Anspruches 1.

Bei der erfindungsgemäßen Vorrichtung handelt es sich um einen vollständig in die Harnröhre versenkbaren Inkontinenzkatheter mit zwei abdichtenden Ballons, wobei sich das den Katheter verschließende Ventil am proximalen, in die Harnblase einragenden Ende des Katheters befindet. Die Öffnung des Ventils erfolgt durch Fingerdruck auf einen am distalen Katheterende und durch die Harnröhrenwand tastbaren, mit Fluid (z.B. Wasser) gefüllten zusätzlichen Ballon, der durch einen in der Katheterwand befindlichen Kanal mit dem am proximalen Katheterende vorhandenen Ventil verbunden ist. Die Konstruktionsmerkmale dieses durch einen hydraulischen Mechanismus auslösbaren Ventils entsprechen keinem bisher bekannten Katheterventil und führen bei geöffnetem Ventil zu keiner Verminderung des Drainagelumens des Katheters, sodaß ein ausreichender Harnstrahl gewährleistet ist. Die Miniaturisierung eines Katheterventils auf die Größe des Innenquerschnittes des Katheters und die damit verbundenen Schwierigkeiten bei der industriellen Herstellung entfallen. Der Verschluß des Ventils erfolgt automatisch durch einen einfachen federelastischen Mechanismus nach Beendigung des Fingerdruckes auf den distalen Katheterballon. Bei hohem Blaseninnendruck erhöht sich zusätzlich die Dichtigkeit des Ventilverschlusses.

Durch die erfindungsgemäße Anbringung des Ventils am proximalen Katheterende ist die Verwendung der Vorrichtung bei der Frau in modifizierter Weise als völlig versenkbarer Inkontinenzkatheter ohne Verbindung zur Körperoberfläche erstmals möglich, wodurch eine aus dem Scheidenvorhof aufsteigende, regelmäßig bei Kathetern oder äußeren Katheterventilen auftretende Entzündung der Harnblase vermeidbar ist.

Das Einführen und Positionieren sowie die Füllung der Katheterballons mit einem Fluid (z.B. Wasser) erfolgt durch einen einmal-verwendbaren, speziellen, sterilen Einführungsstab.

Das Entfernen der Vorrichtung aus der Harnröhre wird durch einen weiteren einmal-verwendbaren, besonders ausgebildeten sterilen Stab bewirkt, durch den der distale Anteil des Katheters abgetrennt wird, wodurch das Fluid (z.B. Wasser) aus den Ballons abfließt. Grundsätzlich kann die Entfernung der Vorrichtung aus der Harnröhre auch unter Sicht durch ein Zystoskop mit Hilfe einer bekannten Fremdkörperzange erfolgen.

Sowohl der männliche als auch der weibliche Inkontinenzkatheter sind somit relativ einfach aufgebaut, die technisch kaum ausführbare Miniaturisierung eines im distalen Katheterlumen untergebrachten Ventils entfällt, sodaß die Inkontinenzkatheter kostengünstig als Einmalartikel herstellbar sind. Zur Produktion bedarf es keiner bisher unbekannten technischen Verfahren. Die in Frage kommenden Werkstoffe sind physiologisch verträgliche Kunststoffe auf Silikon- oder Latexbasis, ggf. mit bekannter hydrophiler Silberbeschichtung zur zusätzlichen Vermeidung einer Keimbesiedelung und mit guter Schleimhautverträglichkeit sowie monofiles chirurgisches Fadenmaterial. Ein Belassen des Katheters in den unteren Harnwegen für mehrere Wochen bis Monate ist deshalb möglich.

Je ein Ausführungsbeispiel für die männliche und weibliche Harnröhre sind nachfolgend anhand der beiliegenden Zeichnungen näher erläutert. In den Zeichnungen zeigen:
FIG. 1 eine schematische Darstellung des männlichen Inkontinenzkatheters mit teilweiser Abbildung des Einführungsstabes,
FIG. 2 eine schematische Darstellung des weiblichen Inkontinenzkatheters mit teilweiser Abbildung des Einführungsstabes,
FIG. 3 eine schematische Darstellung des baugleichen männlichen und weiblichen proximalen Katheterendes bei geschlossenem Ventil in seitlicher Ansicht,
FIG. 4 eine schematische Darstellung des baugleichen männlichen und weiblichen proximalen Katheterendes bei geschlossenem Ventil, mit Sicht auf die der Ventilöffnung gegenüberliegende Seite,
FIG. 5 eine schematische Darstellung des baugleichen männlichen und weiblichen proximalen Katheterendes bei geöffnetem Ventil in seitlicher Ansicht,
FIG. 6 eine schematische Darstellung des erfindungsgemäßen männlichen Inkontinenzkatheters im eingesetzten Zustand in der männlichen Harnröhre, wobei das in die Harnblase einragende Katheterventil geschlossen ist,
FIG. 7 eine schematische Darstellung des erfindungsgemäßen weiblichen Inkontinenzkatheters in der weiblichen Harnröhre bei geöffnetem Ventil,
FIG. 8 eine schematische Darstellung des männlichen Inkontinenzkatheters mit aufgesetztem Einführungsstab in der männlichen Harnröhre während der Füllungsphase der Ballons,
FIG. 9 eine schematische Darstellung des distalen Teiles des männlichen Inkontinenzkatheters in der männlichen Harnröhre im Moment der Ablösung des Einführungsstabes,
FIG. 10 eine schematische Darstellung des distalen Teiles des männlichen Inkontinenzkatheters in der männlichen Harnröhre während des Einführens der speziellen, stabförmigen Entfernungsvorrichtung,
FIG. 11 eine schematische Darstellung des distalen Teiles des männlichen Inkontinenzkatheters in der männlichen Harnröhre zu Beginn der Extraktion durch die Entfernungsvorrichtung, und
FIG. 12 eine schematische Darstellung des distalen Teiles des weiblichen Inkontinenzkatheters in der weiblichen Harnröhre zu Beginn der Extraktion durch die Entfernungsvorrichtung.

In den Zeichnungen sind für Komponenten und Merkmale, die bei den verschiedenen Ausführungsformen gleich sind, gleiche Bezugszeichen verwendet.

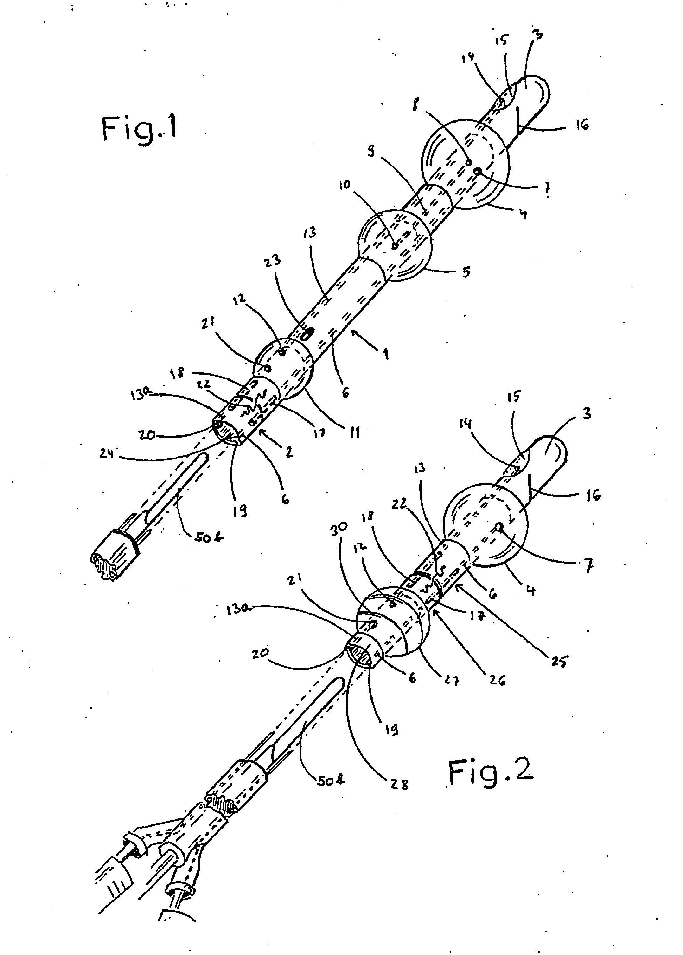

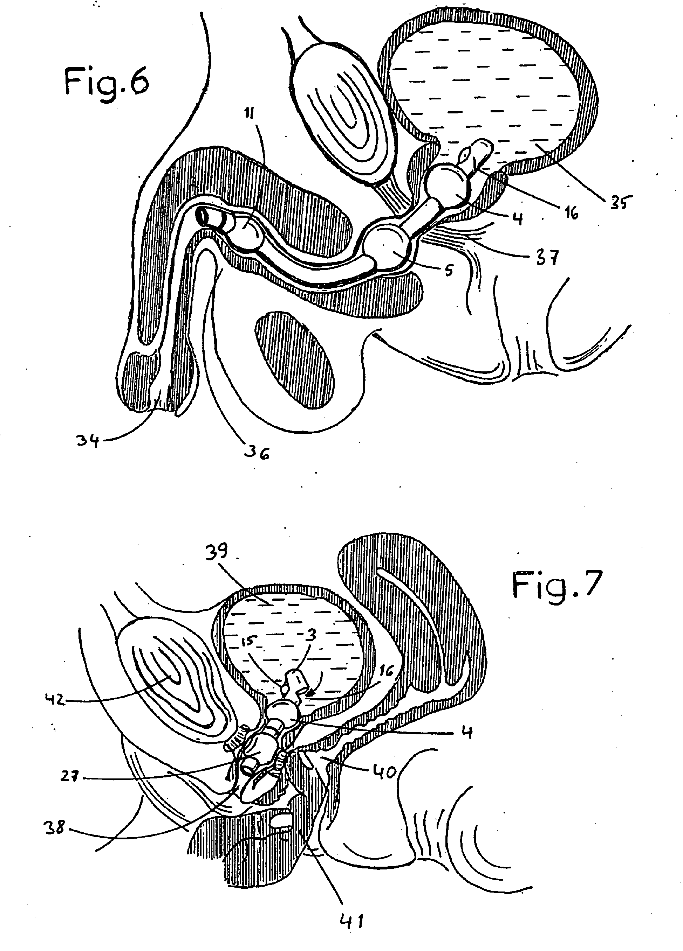

Gemäß Figur 1 besteht die erfindungsgemäße Vorrichtung aus einem schlauch- oder rohrförmigen Katheter, bestehend aus einem proximalen Teil 1 und einem davon lösbaren distalen Teil 2, beides z.B. aus Silikon, dessen Länge so bemessen ist, daß er mit seinem distalen Teil 2 im eingesetztem Zustand innerhalb der männlichen Harnröhre 34 (Fig. 6) liegt und dessen proximales Ende 3 in das Lumen der Harnblase 35 (Fig. 6) einragt. In der Nähe des proximalen Endes 3 ist mit der Außenseite des Katheterteils 1 ein erster Ballon 4 fest verbunden. In einem kurzen Abstand distal davon ist an der Außenseite des Katheterteils 1 ein zweiter (kleinerer) Ballon 5 fixiert. Vom distalen Ende des Katheterteils 2 aus verläuft in dessen und der Wandung des Teils 1 ein gestrichelt dargestellter Kanal 6, der über eine in der Katheterwand vorgesehene Öffnung 7 in das Innere des proximalen Ballons 4 mündet. Neben der Öffnung 7 ist in der Wand des Teils 1 eine weitere, in den Ballon 4 mündende Öffnung 8 ausgebildet, von der aus ein Verbindungskanal 9 (gestrichelt dargestellt) ebenfalls in der Katheterwandung zum mittleren Ballon 5 verläuft und dort in einer Öffnung 10 mündet, die einen deutlich geringeren Querschnitt als die Öffnungen 7 und 8 des proximalen Ballons 4 aufweist. Dadurch erfolgt eine zeitlich verzögerte Füllung des Ballons 5 im Verhältnis zu Ballon 4 bei dem Einbringen eines Fluids (z.B. Wasser) über Kanal 6 in den Ballon 4.

In der Nähe des distalen Endes des Katheterteils 1 befindet sich ein dritter mit der Wandung des Teils 1 verbundener Ballon 11, der durch eine in der Katheterwand befindliche Öffnung 12 in seinem Inneren über einen in der Katheterwand proximal verlaufenden Kanal 13 und eine Öffnung 14 am proximalen Katheterende 3 in einen mit der Wandung des Teils 1 fest verbundenen kleinen Halbballon 15 mündet und der Hauptbestandteil eines Öffnungsmechanismus der Ventilöffnung 16 ist. Die Ventilöffnung 16 ist durch dichtend aneinander liegende Dichtlippen definiert, die durch einen Einschnitt in das Katheterende 3 erzeugt sind und im geschlossenen Zustand durch den Blaseninnendruck noch stärker dichtend aneinander gedrückt werden.

Der distale Katheterteil 2 ist auf den Katheterteil 1 aufgesteckt. Als Steckverbindungen dienen zwei kleinlumige Kunststoffröhrchen 17 und 18. Das Kunststoffröhrchen 17 verbindet dabei den Wandkanal 6 mit einem elastischen Stichventil 19 in der Stirnseite des Teils 2. Das Kunststoffröhrchen 18 setzt einen von einem Stichventil 20 in der Stirnseite des Teils 2 ausgehenden Wandkanal 13a fort, der in der Wandung des Teils 2 verläuft und innerhalb des Ballons 11 in einer Öffnung 21 mündet. Zwei lose Fäden 22 (ein Faden nicht sichtbar), deren eines Ende in der Wandung des dekonnektierbaren distalen Teils 2 und deren anderes Ende in der Wandung des proximalen Teils 1 verankert sind, schaffen eine zusätzliche lose Verbindung zwischen den beiden Katheterteilen 1 und 2. Proximal des distalen Ballons 11 befinden sich eine oder zwei relativ große Öffnungen 23 in der Wandung des Teils 1 zur Drainage von Sekret aus der proximalen Harnröhre in das Lumen 24 des Katheters.

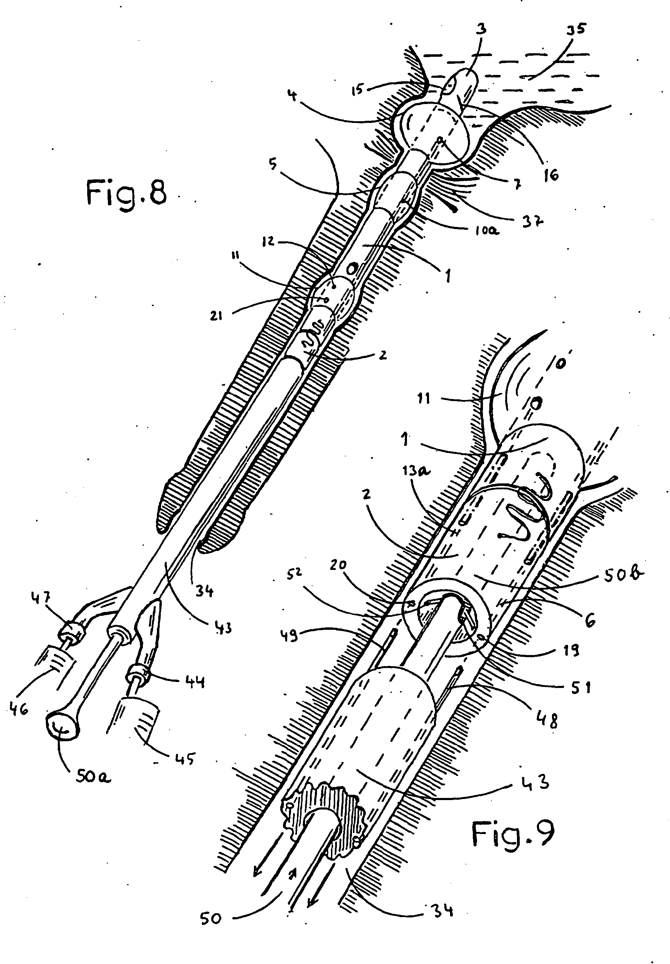

Wie näher aus Fig. 9 hervorgeht und in den Fig. 1 und 2 der Übersichtlichkeit halber nicht dargestellt ist, ist am distalen Ende des Katheterteils 2, quer über dessen Lumen hinweg, ein Faden 52 fest verankert. Der Faden 52 dient, wie weiter unten näher erläutert ist, dem Einsetzen und Entfernen des erfindungsgemäßen Katheters in die bzw. aus der Harnröhre.

Gemäß Fig.2 besteht die erfindungsgemäße, für die Frau bestimmte Vorrichtung aus einem schlauch- oder rohrförmigen Katheter, z.B. aus Silikon, dessen Länge so bemessen ist, daß er mit seinem distalen Ende 28 im eingesetzten Zustand innerhalb der weiblichen Harnröhre 38 (Fig.7) liegt und dessen proximales Ende 3 in das Lumen der Harnblase 39 einragt. Der Katheter besteht aus zwei dekonnektierbaren Teilen, einem proximalen Teil 25 und einem distalen Teil 26.

Mit der Außenseite des proximalen Teils 25 ist ein erster Ballon 4 fest verbunden. Vom distalen Ende 28 des distalen Katheterteils 26 verläuft in dessen Wandung ein Kanal 6, der sich in der Wandung des proximalen Teils 25 fortsetzt und der über eine in dessen Wandung vorgesehene Öffnung 7 in das Innere des proximalen Ballons 4 mündet.

Mit der Wandung des distalen Katheterteils 26 ist ein zweiter Ballon 27 fest verbunden, der durch eine in der Katheterwand vorgesehene Öffnung 12 in seinem Inneren über einen in der Katheterwand befindlichen Kanal 13 und eine Öffnung 14 am proximalen Ende 3 des proximalen Katheterteils 25 in einen mit der Wand des Katheterteils 25 fest verbundenen kleinen Halbballon 15 mündet und der Hauptbestandteil des Öffnungsmechanismus einer Ventilöffnung 16 ist.

Der distale Katheterteil 26 ist auf den proximalen Katheterteil 25 aufgesteckt. Als Steckverbindungen dienen wieder zwei kleinlumige Kunststoffröhrchen 17 und 18. Das Kunststoffröhrchen 17 verbindet dabei den Wandkanal 6 mit einem elastischen Stichventil 19 in der Stirnseite des Teils 26. Das Kunststoffröhrchen 18 verlängert einen von einem Stichventil 20 in der Stirnseite des Teils 26 ausgehenden Wandkanal 13a, der durch eine Öffnung 21 innerhalb des Ballons 27 mündet. Zwei lose Fäden 22 (ein Faden nicht sichtbar), deren eines Ende in der Wandung des distalen Katheterteils 26 und deren anderes Ende in der Wandung des proximalen Katheterteils 25 verankert sind, schaffen eine zusätzliche lose Verbindung zwischen den beiden Katheterteilen 26 und 25.

Um eine Lageänderung der Vorrichtung in der weiblichen Harnröhre 38 bei Betätigung des hydraulischen Auslösemechanismus zum Öffnen der Ventilöffnung 16 durch Kompression des durch die Vorderwand der Scheide 40 (Fig. 7) tastbaren Ballons 27 zu vermeiden, ist der Ballon 27 mit zwei bis vier leistenförmigen zirkulären und quer zur Längsachse der Vorrichtung verlaufenden Vorwölbungen oder Rippen 30 versehen. Die Vorwölbungen 30 können ggf. durch andere die Oberfläche des Ballons 27 verändernde Strukturen (nicht abgebildet) mit gleicher Funktion ersetzt sein.

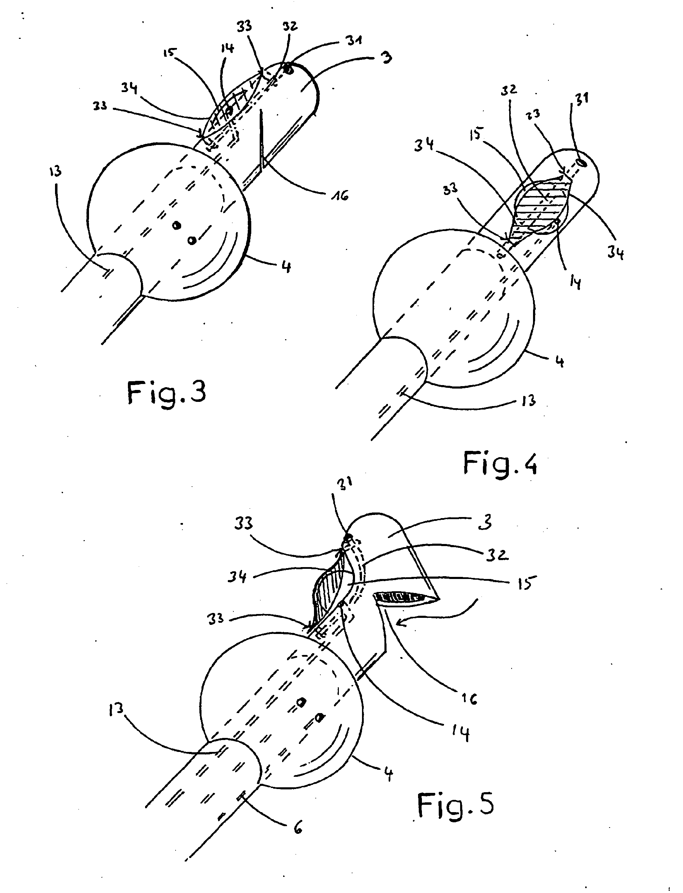

Die Figuren 3, 4 und 5 zeigen Details und die Funktion des am proximalen Katheterende 3 der erfindungsgemäßen männlichen und weiblichen Vorrichtungen in gleicher Weise vorhandenen, in das Katheterlumen drainierenden Ventils.

In die Wandung des proximalen Katheterendes 3 ist unter, das Ventil 16 schließender Vorspannung durch eine Öffnung 31 ein federelastischer Stab aus Metall oder Kunststoff 32 in Längsrichtung versenkt. Dessen Enden sind von zwei Fäden 33 umschlungen, welche ein annähernd rautenförmiges, flach an dem Katheterende 3 und dem Halbballon 15 anliegendes Vlies 34 aus einem gewebeverträglichen Material fixieren. Das Vlies 34 hat eine in seiner Längsmitte verbreiterte Form, um ein Abgleiten von dem Halbballon 15 bei dessen nachfolgend beschriebener Aufwölbung zu vermeiden. Durch Einströmen eines Fluids (z.B. Wasser) unter Druck über den Kanal 13 in der Wandung der Vorrichtung und aus der Öffnung 14 in den Halbballon 15 wird eine Füllung und damit Aufwölbung des kleinen Halbballons 15 bewirkt.

Durch Druck auf den durch die Wand der männlichen Harnröhre 34 (Fig. 6) tastbaren Ballon 11 der männlichen Vorrichtung nach Fig.1 bzw. durch Druck auf den durch die Vorderwand der Scheide 40 (Fig. 7) und die Wand der weiblichen Harnröhre 38 tastbaren Ballon 27 der weiblichen Vorrichtung nach Fig. 2 erhöht sich der Druck eines Fluids (z.B. Wasser) im Kanal 13 der männlichen bzw. der weiblichen Vorrichtung.

Infolge dieser Druckerhöhung wölbt sich der Halbballon 15 auf und durch das den kleinen Halbballon 15 bedeckende, weitgehend undehnbare Vlies 34 wird auf die an dem elastischen Stab 32 befestigten beiden Fäden 33 ein Zug so ausgeübt, daß sich der Kunststoffstab 32 jagdbogenartig zusammen mit der Wandung des proximalen Endes 3 des Katheters in die Richtung des kleinen Halbballons 15 biegt. Dadurch öffnet sich gleichzeitig das an der gegenüberliegenden Seite des proximalen Katheterendes 3 befindliche Ventil 16 ähnlich dem Visier eines mittelalterlichen Ritterhelmes und ermöglicht den Harneinstrom aus der Harnblase 35 bzw. 39. Nach Entleerung der Harnblase 35 bzw. 39 und Aufhebung des Druckes auf den Ballon 11 (Fig. 1) bzw. 27 (Fig. 2) entleert sich der kleine Halbballon 15 durch Rückstrom des Fluids (z.B. Wasser) über Kanal 13 in den Ballon 11 bzw. 27. Vor allem geschieht das durch die federelastische Rückstellkraft des elastischen Stabes 32, da die Biegespannung in diesem aufgehoben wird und dieser seine ursprüngliche Gestalt wieder annimmt. Das proximale Katheterende 3 kehrt dadurch in seine ursprüngliche Form zurück und das Ventil 16 wird unter Druck, verursacht durch den Stab 32, verschlossen.

FIG. 6 zeigt eine schematische Darstellung der Anatomie der männlichen Harnröhre 34 bei voller Harnblase 35 mit in die männliche Harnröhre 34 eingesetzter männlicher Vorrichtung nach Fig. 1, wobei die in die Harnblase einragende Ventilöffnung 16 geschlossen ist. Der zur hydraulischen Öffnung der Ventilöffnung 16 dienende Ballon 11 liegt etwa in der Höhe des Skrotalansatzes 36. Die abdichtenden Ballons 4 und 5 der Vorrichtung sind oberhalb und unterhalb des Bereiches des Schließmuskels 37 in gefülltem Zustand dargestellt.

FIG. 7 zeigt eine schematische Darstellung der Anatomie der weiblichen Harnröhre 38 bei voller Harnblase 39 mit in die Harnröhre 38 eingesetzter weiblicher Vorrichtung nach Fig.2, deren proximaler Teil 25 (Fig.2) mit dem abdichtenden Ballon 4 und dem proximalen Ende 3 in die volle Harnblase 39 einragt. Der in die äußere Öffnung der Scheide 40 eingeführte Zeigefinger 41 komprimiert den durch die vordere Wand der Scheide 40 und die hintere Wand der Harnröhre 38 tastbaren Ballon 27 der Vorrichtung, wobei die Symphyse 42, die Teil des knöchernen Beckenringes ist, als Widerlager dient. Dadurch ist der Halbballon 15 des proximalen Katheterendes 3 aufgewölbt und das Ventil 16 geöffnet, wodurch der Harn in das Lumen der Vorrichtung in Pfeilrichtung einströmt.

FIG. 8 zeigt in schematischer Darstellung die gerade in die männliche Harnröhre 34 eingeführte aber noch nicht festgelegte männliche Vorrichtung mit einem speziellen, starren oder teilelastischen, sterilen aufgesteckten Füll- und Einführungsstab 43, wobei das proximale Ende 3 in die Harnblase 35 mit entleertem Halbballon 15 und damit geschlossenem Ventil 16 einragt. Der abdichtende Ballon 4 des proximalen Teils 1 ist durch Fluid (z.B. Wasser) mit Hilfe einer auf ein Ventil 44 des Einführungsstabes 43, das einem bekannten Katheterventil entspricht, aufgesetzten Spritze 45 bereits gefüllt. Der zweite abdichtende Ballon 5 des proximalen Katheterteils 1 ist dabei noch nicht entfaltet.

Dabei ist eine mögliche vereinfachende Konstruktionsvariante der männlichen Vorrichtung angedeutet, die darin besteht, daß Ballon 4 und Ballon 5 unter Wegfall des Kanals 9 durch einen einzigen in der Wandung der Vorrichtung vorhandenen Kanal 6 (Fig. 1) geradlinig miteinander verbunden sind, wobei die einzige Öffnung 7 in der Katheterwandung innerhalb des Ballons 4 einen deutlich größeren Querschnitt aufweist als eine mit dem Kanal 6 verbundene Öffnung 10a des Ballons 5, so daß nach Füllung des Ballons 4 ein kurzer Zeitraum bleibt, um die Vorrichtung durch Zurückziehen derselben unter leichtem Zug am Einführungsstab 43 in die regelrechte Position zu bringen. Dabei kommt der Ballon 4 oberhalb der, auch bei Inkontinenten noch vorhandenen, naturgegebenen Enge der Harnröhre 34 im Bereich des Schließmuskels 37 zu liegen. Erst dann füllt sich, wie bei der Konstruktion in Fig. 1 dargestellt, durch die drosselnde Wirkung der Öffnung 10a der Ballon 5 mit Fluid (z.B. Wasser). Die in Fig. 1 dargestellte zusätzliche Wandöffnung 8 innerhalb des Ballons 4 und der entsprechende Kanal 9 sind nicht vorhanden.

Der Ballon 11 wird nach Erreichen der richtigen Position der Vorrichtung wie beschrieben in der männlichen Harnröhre 34 mit Hilfe einer Spritze 46 über ein mit Ventil 44 baugleiches Ventil 47 durch den Führungsstab 43 mit wenigen Millilitern Fluid (z.B. Wasser) gefüllt. Um eine zeitgleiche maximale Füllung des Halbballons 15 am proximalen Ende 3 der Vorrichtung zu vermeiden, können die Öffnungen 21 und 12 in der Wandung des proximalen Katheterteils 1 innerhalb des Ballons 11 einen unterschiedlichen Querschnitt der Art aufweisen, daß die Öffnung 12 einen etwas geringeren Querschnitt als die Öffnung 21 hat.

FIG. 9 zeigt in schematischer Darstellung eine Teilansicht des distalen Teils der männlichen Vorrichtung nach Fig. 1 in der männlichen Harnröhre 34 im Moment der Ablösung des Einführungsstabes 43 vom distalen Teil 2 der Vorrichtung, nachdem die Ballons 4,5 und 11 (Fig. 8 und Fig. 1) nach Injektion von Fluid (z.B. Wasser) über die Ventile 44 und 47 durch kanülenartige, mit runder Spitze versehene Fortsätze 48 und 49 des Führungsstabes 43 und durch die elastischen Stichventile 19 und 20 im distalen Teil 2 der Vorrichtung bereits gefüllt sind. (Ballon 4 und 5 nicht dargestellt)

In dem Einführungsstab 43 ist ein in Längsrichtung verschieblicher, stabförmiger Kern 50 geführt, der mit seinem vorderen Endabschnitt 50b in das Lumen des Katheters so weit einführbar ist, daß er die Trennstelle zwischen den Teilen 1 und 2 übergreift (in Fig.9 gestrichelt gezeigt). Hierdurch soll beim Einführvorgang des Katheters die Trennstelle versteift und sollen die die Trennstelle überbrückenden Röhrchen 17,18 vor einer Verbiegung geschützt werden. Im Ausführungsbeispiel ist der vordere Endabschnitt 50b auf einen halbkreisförmigen Querschnitt abgesetzt, um den im Lumen des distalen Teils 2 der Vorrichtung querverlaufenden und mit der Wandung des distalen Teils 2 der Vorrichtung fest verankerten Faden 52 passieren zu können. Am hinteren Ende des Endabschnitts 50b weist die durch diesen gebildete Schulter eine Einkerbung 51 auf, die den Faden 52 aufnimmt. Eine Dislokation der Vorrichtung bei der Extraktion des Führungsstabes 43 auf Grund einer, zu Beginn der Positionierung der Vorrichtung in der männlichen Harnröhre 34 notwendigen, Haftung der Kanülen 48 und 49 des Führungsstabes 43 in den Stichventilen 19 und 20 wird durch leichten Druck auf das äußere Ende 50 a des Kernstabes 50 vermieden.

Die Positionierung der weiblichen Vorrichtung nach Fig. 2 in der weiblichen Harnröhre 38, Fig. 7 erfolgt in gleicher Weise (nicht bildlich dargestellt) mit Hilfe eines baugleichen, ggf. etwas kürzeren Einführungsstabes 43 nach Fig. 8.

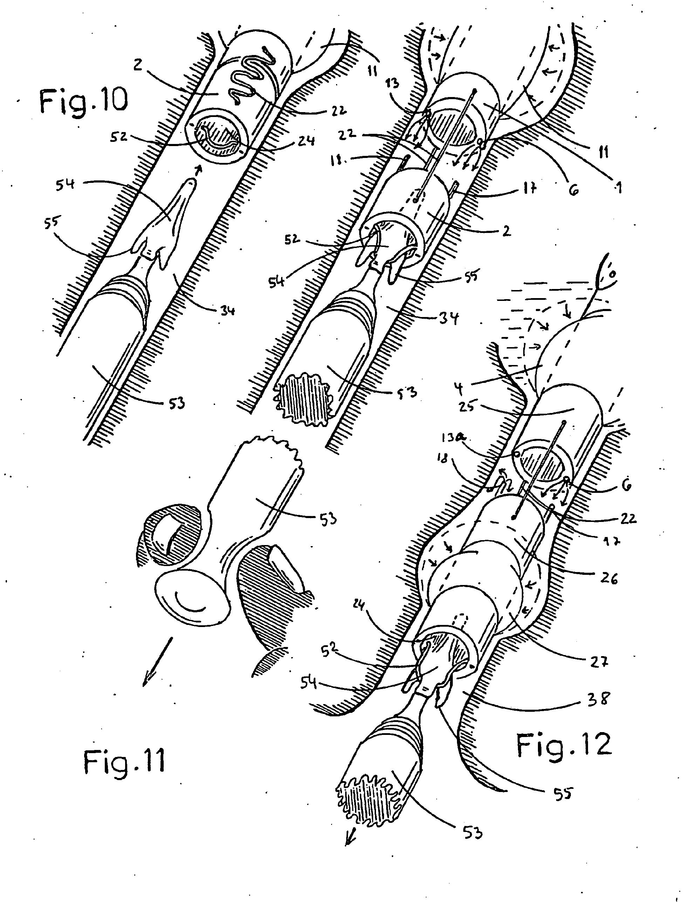

Fig. 10 und 11 zeigen in schematischer Darstellung eine Teilansicht der männlichen Vorrichtung nach Fig. 1 während des Vorganges der Entfernung aus der männlichen Harnröhre 34. Dabei wird ein spezieller starrer oder teilelastischer steriler Extraktionsstab 53 mit seinem Ende, das mit einem abgerundeten, harpunenartigen Fortsatz 54 versehen ist, blind zunächst in die männliche Harnröhre 34 und dann in das offene Lumen 24 des distalen Teiles 2 der Vorrichtung vorgeschoben. Beim anschließenden Zurückziehen des Extraktionsstabes 53 nimmt einer von zwei oder mehreren abgerundeten Widerhaken 55 des Fortsatzes 54 den quer zum Lumen des distalen Teiles 2 und fest in dessen Wandung verankerten Faden 52 auf. Durch weiteren Zug am Extraktionsstab 53 (durch einen Pfeil ist in Fig. 11 die Zugrichtung dargestellt) wird der distale Teil 2 vom proximalen Teil 1 der Vorrichtung gelöst. Dabei werden die beiden Kunststoffröhrchen 17 und 18 aus den Lumina der Wandkanäle 6 und 13 extrahiert, wodurch sich die Ballons 4, 5 und 11 über die dadurch frei werdenden Öffnungen der Kanäle 6 und 13 in der Wandung des proximalen Teils 1 der Vorrichtung in das Lumen der Harnröhre 34 (durch Pfeile dargestellt) entleeren. Die Teile 1 und 2 bleiben durch die beiden sich spannenden Fäden 22, die fest in den Wandungen der Teile 1 und 2 verankert sind, verbunden. Durch weiteren Zug am Extraktionsstab 53 läßt sich nun die gesamte Vorrichtung leicht aus der männlichen Harnröhre 34 extrahieren.

Fig. 12 zeigt in schematischer Darstellung eine Teilansicht der weiblichen Vorrichtung nach Fig. 2 während des Vorgangs der Extraktion aus der weiblichen Harnröhre 38. Nach Einführen eines mit nach Fig. 10 und 11 grundsätzlich baugleichen, ggf. kürzeren Extraktionsstabes 53 in die weibliche Harnröhre 38 und dessen Spitze 54 in das distal offene Lumen 24 des distalen Teils 26 der Vorrichtung verfangen sich die abgerundeten Widerhaken 55 des harpunenartigen Fortsatzes 54 des Extraktionsstabes 53 in dem im Lumen 24 des distalen Teils 26 vorhandenen Faden 52. Durch Zurückziehen des Extraktionsstabes 53 wird der distale Teil 26 vom proximalen Teil 25 getrennt. Die beiden mit Fluid gefüllten Ballons 27 und 4 entleeren sich über das den Wandkanal 13 teilweise bildende, mit Teil 26 der Vorrichtung fest verbundene Kunststoffröhrchen 18 bzw. das dadurch gleichzeitig offene Lumen des Wandkanals 6 des proximalen Teils 25 der Vorrichtung (durch Pfeile dargestellt) in die weibliche Harnröhre 38. Durch weiteren Zug am Extraktionsstab 53 (durch Pfeil ist die Zugrichtung dargestellt) wird die weibliche Vorrichtung aus der weiblichen Harnröhre 38 entfernt.

Grundsätzlich kann der Vorgang der Extraktion der männlichen Vorrichtung nach Fig. 1 aus der männlichen Harnröhre 34, Fig. 10 und 11 und der weiblichen Vorrichtung nach Fig.2 aus der weiblichen Harnröhre 38, Fig. 12 auch unter Sicht mit einem Zystokop mit Hilfe einer bekannten Fremdkörperzange erfolgen.

Die für die Extraktion erforderliche Entleerung der Ballons 4,5 und 11 bzw. 4 und 27 kann auch über eine modifizierte Konstruktion, die in den Zeichnungen nicht dargestellt ist, erreicht werden. Bei dieser Konstruktion ist vorgesehen, daß die zugehörigen Kanäle 6 bzw. 13 über je ein zum Lumen des Katheters gerichtetes, zweckmäßigerweise am distalen Ende des Katheters angebrachtes "Fenster" zugänglich sind, das zum Zweck der Extraktion gezielt zerstört werden kann. Dieses Fenster kann eine bis zum Zeitpunkt der Zerstörung mittels einer Sonde oder Fremdkörperzange, durch eine Membran verschlossene Öffnung sein.

Im Rahmen der Erfindung kann von den vorstehend beschriebenen Ausführungsbeispielen abgewichen werden. So ist die Erfindung nicht auf die durch einen Einschnitt realisierte Ausführungsform des Ventils 16 an dem Katheterende 3 beschränkt. Vielmehr sind alle Ventile denkbar, die durch eine hydraulische Betätigung an dem Katheterende 3 eine Eintrittsöffnung freigeben. Weiterhin kann daran gedacht werden, das als Übertragungselement dienende Vlies 34, welches durch Biegeverformung des federelastischen Stabes 32 das Ventil 16 öffnet, durch eine Membran oder dergleichen vor nachteiligen Einwirkungen des Harns zu schützen. Eine solche Membran kann das Katheterende 3 weitgehend im Bereich des Vlieses 34 umschließen, ohne jedoch eine mechanische Rückwirkung darauf auszuüben. Anstelle des Vlieses 34 kann jegliches chirurgische Material eingesetzt werden, das imstande ist, eine Zugkraft ohne merkliche elastische Dehnung auf den federelastischen Stab 32 zu übertragen.

Es ist weiterhin auch nicht zwingend erforderlich, den Betätigungsballon 11 bzw. 27, durch den der Halbballon 15 des Betätigungsmechanismus zur Aufwölbung gebracht wird, erst nach dem Einsetzen des Katheters mit Fluid aufzufüllen. Das Fluid kann vielmehr von vornherein in dem geschlossenen System enthalten sein, das durch den Betätigungsballon 11 bzw. 27, den Kanal 13 und den Halbballon 15 gebildet ist.

## Patentansprüche

1. Vorrichtung zur Behandlung von Harnblasen-Entleerungsstörungen des Menschen, mit einem in die Harnröhre einführbaren Katheter (1, 2), der zur Abdichtung der Harnblase und zur Halterung des Katheters im Blasenlumen eine mit Fluid auffüllbare Ballonanordnung (4, 5 bzw. 4, 27) trägt, die durch mindestens einen längs der Katheterwand verlaufenden und am distalen Endabschnitt des Katheters verschlossenen Kanal (6, 9, 13a) mit dem Fluid auffüllbar bzw. entleerbar ist, und mit einem im proximalen, im eingesetzten Zustand des Katheters (1, 2) im Blasenlumen liegenden Endabschnitt (3) des Katheters untergebrachten selbsttätig schließenden Ventil (16), wobei die Länge des Katheters (1, 2) derart bemessen ist, dass sein distales Ende im eingesetzten Zustand innerhalb der Harnröhre liegt und das Ventil (16) durch einen Betätigungsmechanismus außerhalb der Harnröhre hydraulisch betätigbar ist, **dadurch gekennzeichnet, dass** der Betätigungsmechanismus (15, 32, 34) zum Öffnen des Ventils (16) hydraulisch betätigt ist und dass der Betätigungsmechanismus (15, 32, 34) durch mechanischen Druck auf einen am distalen Endabschnitt des Katheters (1, 2) angeordneten, mit Fluid gefüllten und über einen Verbindungskanal (13) mit dem Betätigungsmechanismus verbundenen Betätigungsballon (11, 27) hydraulisch beaufschlagbar ist.

2. Vorrichtung nach Anspruch 1, **dadurch gekennzeichnet, daß** das Ventil (16) ein elastisch rückstellbares Klappventil ist, das durch ein Betätigungselement (15) des Betätigungsmechanismus von seinem Ventilsitz abhebbar ist.

3. Vorrichtung nach Anspruch 2, **dadurch gekennzeichnet, daß** das Klappventil eine seitliche Schlitzöffnung (16) in dem proximalen Endabschnitt (3) ist, deren Schlitzränder Dichtlippen bilden.

4. Vorrichtung nach Anspruch 2 oder 3, **dadurch gekennzeichnet, daß** der Betätigungsmechanismus ein von dem Betätigungselement (15) auslenkbares weitgehend undehnbares Zugelement (34) umfaßt.

5. Vorrichtung nach Anspruch 4, **dadurch gekennzeichnet, daß** das Betätigungselement eine hydraulisch aufweitbare Membran (15) ist, deren eine Seite über den Verbindungskanal (13) mit Hydraulikdruck beaufschlagbar ist und durch deren Aufweitung das gespannte Zugelement (34) auslenkbar ist.

6. Vorrichtung nach einem der Ansprüche 2 bis 5, **dadurch gekennzeichnet, daß** das Klappventil (16) durch einen federelastischen Abschnitt (32) des proximalen Endabschnitts (3) schließbar ist.

7. Vorrichtung nach Anspruch 6, **dadurch gekennzeichnet, daß** der federelastische Abschnitt durch einen in der Katheterwandung vorgesehenen biegeelastischen Federstab (32) gebildet ist.

8. Vorrichtung nach Anspruch 4 und einem der Ansprüche 5 bis 7, **dadurch gekennzeichnet, daß** das Zugelement (34) ein die aufweitbare Membran straff überdeckendes Zugband ist, dessen Enden jeweils mit den Dichtlippen des Ventils (16) zugeordneten Bereichen des proximalen Endabschnitts (3) fest verbunden sind.

9. Vorrichtung nach einem der Ansprüche 1 bis 8, **dadurch gekennzeichnet, daß** der Betätigungsmechanismus (15,32,34) durch eine Schutzmembran umschlossen ist.

10. Vorrichtung nach einem der Ansprüche 1 bis 9, **dadurch gekennzeichnet, daß** der Betätigungsballon (11,27) durch einen am distalen Endabschnitt des Katheters verschlossenen Kanal (13a) mit Fluid auffüllbar ist.

11. Vorrichtung nach einem der Ansprüche 1 bis 10, **dadurch gekennzeichnet, daß** die Ballonanordnung zur Abdichtung der Harnblase und Halterung des Katheters im Blasenlumen bei der Vorrichtung für den Mann durch einen ersten Ballon (4) an dem proximalen Endabschnitt (3) und einen distal von dem ersten Ballon (4) angeordneten zweiten Ballon (5) gebildet ist, der im eingesetzten Zustand des Katheters außerhalb des Blasenschließmuskels liegt.

12. Vorrichtung nach einem der Ansprüche 1 bis 10, **dadurch gekennzeichnet, daß** die Ballonanordnung zur Abdichtung der Harnblase und Halterung des Katheters im Blasenlumen bei der Vorrichtung für die Frau durch einen ersten Ballon (4) an dem proximalen Endabschnitt (3) und den Betätigungsballon (27) gebildet ist.

13. Vorrichtung nach Anspruch 12, **dadurch gekennzeichnet, daß** der Betätigungsballon (27) auf seiner Außenseite Vorsprünge zur Lagefixierung aufweist.

14. Vorrichtung nach einem der Ansprüche 1 bis 13, **dadurch gekennzeichnet, daß** der Katheter aus einem proximalen Teil (1) und einem distalen Teil (2) besteht, die durch eine Steckverbindung (17,18) miteinander lösbar verbunden und durch eine nach Lösung der Steckverbindung wirksam werdende zugfeste Verbindung (22) aneinander befestigt sind.

15. Vorrichtung nach Anspruch 14, **dadurch gekennzeichnet, daß** die Steckverbindung von dem proximalen Stirnende des distalen Teils (2) vorspringende Röhrchen (17,18) umfaßt, die in Mündungen der Kanäle (6,13,13a) am distalen Ende des proximalen Teils (1) einsteckbar sind und eine Fortsetzung dieser Kanäle zum distalen Ende des Katheters (1,2) bilden.

16. Vorrichtung nach Anspruch 14 oder 15, **dadurch gekennzeichnet, daß** die zugfeste Verbindung zwischen dem proximalen Teil (1) und dem distalen Teil (2) durch einen Faden (22) gebildet ist, dessen Länge die Lösung der Steckverbindung (17,18) erlaubt.

17. Vorrichtung nach einem der Ansprüche 14 bis 16, **dadurch gekennzeichnet, daß** an dem distalen Ende des distalen Teils (2) ein Einhängelement (52) befestigt ist, das bei der Extraktion der Vorrichtung durch ein Extraktionsinstrument (53,54,55) hintergriffen werden kann.

18. Vorrichtung nach Anspruch 17, **dadurch gekennzeichnet, daß** das Einhängelement durch einen quer über die distale Katheteröffnung gespannten Faden (52) gebildet ist, der bei der Einführung der Vorrichtung zugleich ein Anlageelement für eine Einführvorrichtung ist.

19. Vorrichtung nach einem der Ansprüche 14 bis 18, **dadurch gekennzeichnet, dass** sie einen ihr Einführen in die männliche Harnröhre ermöglichenden, auf das distale Ende des distalen Teils (2) aufsteckbaren Schaft (43) aufweist, in dem ein stabförmiger Kern (50) verschiebbar geführt ist, dessen vorderer Endabschnitt (50b) zur Anlage an dem distalen Ende des Katheters aus dem Schaft (43) ausschiebbar ist.

20. Vorrichtung nach Anspruch 19, **dadurch gekennzeichnet, dass** der vordere Endabschnitt (50b) des Kerns (50) bis zu einem Anschlag (51) in das Lumen des Katheters einführbar ist und im eingeführten Zustand eine Trennstelle des aus einem proximalen und einem distalen Teil (1 bzw. 2) bestehenden Katheters versteifend übergreift.

21. Vorrichtung nach Anspruch 20, **dadurch gekennzeichnet, dass** der vordere Endabschnitt (50b) im Querschnitt gegenüber dem Kern (50) verringert ist und der Anschlag eine Schulter (51) ist.

22. Vorrichtung nach einem der Ansprüche 19 bis 21, **dadurch gekennzeichnen, dass** das hintere Ende (50a) des stabförmigen Kerns (50) als Betätigungsende aus dem Schaft (43) herausragt.

23. Vorrichtung nach einem der Ansprüche 19 bis 22, **dadurch gekennzeichnet, dass** die Einführvorrichtung zugleich als Füllvorrichtung zum Auffüllen der Ballone mit Fluid ausgebildet ist, indem das vordere Ende des Schafts (43) als Steckverbindung mit in stirnseitige Stichventile (19, 20) des Katheterteils (2) einschiebbaren Kanülen (48, 49) ausgestattet ist, welche über in dem Schaft (43) verlaufende Zuführkanäle mit an dem Schaft (43) ausgebildeten Anschlüssen (44, 47) verbunden sind.

24. Vorrichtung nach einem der Ansprüche 1 bis 18, **dadurch gekennzeichnet, dass** in dem Lumen des Katheters (1, 2) ein Einhängeelement (52) vorgesehen ist und dass ein eine Extraktion der Vorrichtung ermöglichender, in die Harnröhre einführbarer Extraktionsstab (53), an dessen vorderen Ende ein mindestens einen Widerhaken (55) aufweisender Fortsatz (54) ausgebildet ist, der in das Lumen des Katheters (1, 2) einführbar ist, mit dem Widerhaken (55) in das dort vorgesehene Einhängeelement einhakbar ist.

## Claims

1. Device for the treatment of human urinary incontinence comprising
a catheter (1, 2) which can be inserted into the urethra and carries a balloon arrangement (4, 5 or 4, 27) which can be filled with fluid to close off the urinary bladder and to hold the catheter in the lumen of the urethra, which fluid can be admitted to and discharged from this balloon arrangement via at least one channel (6, 9, 13a) extending along the catheter wall which is closed off at the distal end of the catheter, and
a self-closing valve (16) mounted at a proximal end section (3) of the catheter, which end section being situated in the lumen of the urethra when the catheter is in its inserted condition,
whereby the length of of the catheter (1, 2) is dimensioned such that its distal end in the inserted condition lies within the urethra and the valve (16) can be actuated by an actuating mechanism (15, 32, 34) outside thr urethra,
**characterized in that** the actuating mechanism (15, 32, 34) is operated hydraulically for opening the valve (16) and that the actuating mechanism (15, 32, 34) can be hydraulically pressurized by mechanical pressure exerted on an actuating balloon (11, 27) located at the distal end section of the catheter (1, 2) and which is filled with fluid and connected to the actuating mechanism via a connecting channel (13).

2. Device in accordance with claim 1, **characterized in that** the valve (16) is an elastically resilient flap valve which can be lifted from its valve seat by an actuating element (15) of the actuating mechanism.

3. Device in accordance with claim 2, **characterized in that** the flap valve (16) is a lateral slit opening (16) in the proximal end section (3), the edges of which form sealing lips.

4. Device in accordance with claim 2 or 3, **characterized in that** the actuating mechanism comprises a substantial non-elastic traction element (34) which is deflectable by the actuating element (15).

5. Device in accordance with claim 4, **characterized in that** the actuating element is a hydraulically expandable membrane (15), one side of which can be hydraulically pressurized via the connecting channel (13) and through the expansion of which the tensioned traction element (34) is deflectable.

6. Device in accordance with one of the claims 2 to 5, **characterized in that** the flap valve (16) can be closed off by a resilient elastic section (32) of the proximal end section (3).

7. Device in accordance with claim 6, **characterized in that** the resilient elastic section is formed by a flexible elastic spring rod (32) located in the catheter wall.

8. Device in accordance with claim 4 and one the claims 5 to 7, **characterized in that** the traction element (34) is a traction band tightly covering the expandable membrane, the ends of which are permanently connected to the areas of the proximal end section (3) associated to the sealing lips of the valve (16).

9. Device in accordance with one of the claims 1 to 8, **characterized in that** the actuating mechanism (15, 32, 34) is surrounded by a protective membrane.

10. Device in accordance with one of the claims 1 to 9, **characterized in that** the actuating balloon (11, 27) can be filled with fluid via a closed channel (13a) at the distal and of the catheter.

11. Device in accordance with one of the claims 1 to 10, **characterized in that** the balloon arrangement for closing off the urinal bladder and holding the catheter in the bladder lumen in the male device is formed by a first balloon (4) at the proximal end (3) and a second balloon (5) located distally from the first balloon (4), which with the catheter in inserted condition lies outside the bladder sphincter muscle.

12. Device in accordance with one of the claims 1 to 10, **characterized in that** the balloon arrangement for closing off the urinal bladder and holding the catheter in the bladder lumen in the female device is forrned by a first balloon (4) at the proximal end (3) and the actuating balloon (27).

13. Device in accordance with claim 12, **characterized in that** the actuating balloon (27) has protrusions on its outer surface for fixing its position.

14. Device in accordance with one of the claims 1 to 13, **characterized in that** the catheter consists of a proximal part (1) and a distal part (2) which are linked separably by a plugged connection (17, 18) and are connected together by a tensile connection (22) after separation of the plugged connection.

15. Device in accordance with claim 14, **characterized in that** the plugged connection comprises small pipes (17, 18) extending from the proximal face end of the distal part (2) and being insertable into openings of the channels (6, 13, 13a) at the distal end of the proximal part (1) and forming a continuation of these channels to the distal end of the catheter (1, 2).

16. Device in accordance with claim 14 or 15, **characterized in that** the tensile connection between the proximal part (1) and the distal part (2) is formed by a thread (22) whose length permits the separation of the plugged connection (17, 18).

17. Device in accordance with one of the claims 14 to 16, **characterized in that** a hooking element (52) is attached to the distal end of the distal part (2) in which an extraction instrument (53, 54, 55) can engage during extraction of the device.

18. Device in accordance with claim 17, **characterized in that** the hooking element is formed by a threard (52) extending cross-wise over the distal catheter opening which, at the same time, forms a contact element for an insertion device during insertion of the catheter.

19. Device in accordance with one of the claims 14 to 18, **characterized in that** it includes a rod-like core (50) for the enabling of its insertion into the male urethra which core is shiftably located in the said shaft (43) and the front of which (50b) can be pushed out of the shaft (43) to contact the distal end of the catheter.

20. Device in accordance with claim 19, **characterized in that** the front end section (50b) of the core (50) can be inserted into the lumen of the catheter up to a stop (51) and in inserted condition reinforcingly bridges a joint in the catheter consisting of a proximal part and a distal part (1 or 2).

21. Device in accordance with claim 20, **characterized in that** the cross-section of the front end section (50b) is smaller in respect to the core (50) and that the stop is a shoulder (51).

22. Device in accordance with one of the claims 19 to 21, **characterized in that** the rear end (50a) of the rod-like core (50) protrudes from the shaft (43) as an actuating end.

23. Device in accordance with one of the claims 19 to 22, **characterized in that** the insertion device is, at the same time, designed as a filling device to allow the balloons to be filied with fluid **in that** the front end of the shaft (43) is designed as a plug connection with canulae (48, 49) which can be pushed into puncture valves (19,20) in the face end of catheter part (2) and which are connected to connectors (44, 47) being formed at the shaft (43) via leading channels formed within the shaft (43).

24. Device in accordance with one of the claims 1 to 18, **characterized in that** a hooking element (52) is provided in the lumen of the catheter (1, 2) and that an extraction rod (53) which enables an extraction of the device is insertable into the urethra, whereby an extension element (54) having at least one barb is formed at the front end of said extraction rod and being insertable into the lumen of the catheter (1, 2) in which the said extension element is hookable with the barb (55) into the hooking element provided there.

## Revendications

1. Dispositif de traitement de troubles d'évacuation de la vessie de l'être humain, comportant un cathéter (1, 2) pouvant être introduit dans l'urètre, lequel cathéter, pour étanchéifier la vessie et pour maintenir le cathéter dans le lumen de la vessie, supporte un dispositif de ballon (4, 5 ou 4, 27) pouvant être rempli de fluide qui peut être rempli ou vidé de fluide par au moins un canal (6, 9, 13a) s'étendant le long de la paroi du cathéter et fermé au niveau de la section terminale distale du cathéter, et une soupape (16) se fermant automatiquement logée, en état posé du cathéter (1, 2) dans la section terminale (3) située dans le lumen de la vessie du cathéter, la longueur du cathéter (1, 2) étant fixée de manière à ce que son extrémité distale, en état posé, se situe à l'intérieur de l'urètre et la soupape (16) étant actionnable par un mécanisme d'actionnement en dehors de l'urètre, **caractérisé en ce que** le le mécanisme d'actionnement (15, 32, 34) permettant d'ouvrir la soupape (16) étant actionné par système hydraulique et **en ce que** le mécanisme d'actionnement (15, 32, 34) peut être sollicité par système hydraulique par une pression mécanique sur un ballon d'actionnement (11, 27) disposé au niveau de la section terminale distale du cathéter (1, 2), rempli de fluide et relié par un canal de liaison (13) au mécanisme d'actionnement.

2. Dispositif selon la revendication 1, **caractérisé en ce que** la soupape (16) est un clapet de fermeture à rappel élastique qui peut être levé de son siège de soupape par un élément d'actionnement (15) du mécanisme d'actionnement.

3. Dispositif selon la revendication 2, **caractérisé en ce que** le clapet de fermeture est une fente d'ouverture latérale (16) de la section terminale proximale (3) dont les bords de fente forment des lèvres d'étanchéité.

4. Dispositif selon la revendication 2 ou 3, **caractérisé en ce que** le mécanisme d'actionnement comprend un élément de traction inextensible dans une large mesure (34) et pouvant être dévié de l'élément d'actionnement (15).

5. Dispositif selon la revendication 4, **caractérisé en ce que** l'élément d'actionnement est une membrane (15) pouvant être élargie par système hydraulique dont une face peut être sollicitée par pression hydraulique par le biais du canal de liaison (13) et à travers l'élargissement de laquelle l'élément de traction tendu (34) peut être dévié.

6. Dispositif selon l'une quelconque des revendications 2 à 5, **caractérisé en ce que** le clapet de fermeture (16) peut être fermé par une section à élasticité de ressort (32) de la section terminale proximale (3).

7. Dispositif selon la revendication 6, **caractérisé en ce que** la section à élasticité de ressort est constituée par une tige à ressort (32) à flexion élastique prévue dans la paroi du cathéter.

8. Dispositif selon la revendication 4 et l'une quelconque des revendications 5 à 7, **caractérisé en ce que** l'élément de traction (34) est un tirant recouvrant de manière serrée la membrane élargissable et dont les extrémités sont reliées fixement respectivement aux zones associées aux lèvres d'étanchéité de la soupape (16) de la section terminale proximale (3).

9. Dispositif selon l'une quelconque des revendications 1 à 8, **caractérisé en ce que** le mécanisme d'actionnement (15, 32, 34) est entouré d'une membrane protectrice.

10. Dispositif selon l'une quelconque des revendications 1 à 9, **caractérisé en ce que** le ballon d'actionnement (11, 27) peut être rempli de fluide par un canal (13a) fermé au niveau de la section terminale distale du cathéter.

11. Dispositif selon l'une quelconque des revendications 1 à 10, **caractérisé en ce que** le dispositif de ballon est constitué, pour étanchéifier la vessie et maintenir le cathéter dans le lumen de la vessie dans le dispositif destiné aux hommes, d'un premier ballon (4) au niveau de la section terminale proximale (3) et d'un second ballon (5) disposé de manière distale par rapport au premier ballon (4) qui, en état posé du cathéter, se trouve en dehors du sphincter de la vessie.

12. Dispositif selon l'une quelconque des revendications 1 à 10, **caractérisé en ce que** le dispositif de ballon est constitué, pour étanchéifier la vessie et maintenir le cathéter dans le lumen de la vessie dans le dispositif destiné aux femmes, d'un premier ballon (4) au niveau de la section terminale proximale (3) et d'un ballon d'actionnement (27).

13. Dispositif selon la revendication 12, **caractérisé en ce que** le ballon d'actionnement (27) présente sur sa face extérieure des saillies pour le fixer en position.

14. Dispositif selon l'une quelconque des revendications 1 à 13, **caractérisé en ce que** le cathéter est composé d'un élément proximal (1) et d'un élément distal (2) qui sont reliés entre eux de manière dissociable par un emboîtement (17, 18) et sont fixés l'un à l'autre par un raccord (22) résistant à la traction et devenant actif après dissociation de l'emboîtement.

15. Dispositif selon la revendication 14, **caractérisé en ce que** l'emboîtement comprend des canules (17, 18) saillant de l'extrémité frontale proximale de l'élément distal (2), pouvant être fichées dans des embouchures des canaux (6, 13, 13a) au niveau de l'extrémité distale de l'élément proximal (1) et constituant un prolongement de ces canaux vers l'extrémité distale du cathéter (1, 2).

16. Dispositif selon la revendication 14 ou 15, **caractérisé en ce que** le raccord résistant à la traction entre l'élément proximal (1) et l'élément distal (2) est constitué par un fil (22) dont la longueur permet de dissocier l'emboîtement (17, 18).

17. Dispositif selon l'une quelconque des revendications 14 à 16, **caractérisé en ce que,** à l'extrémité distale de l'élément distal (2), est fixé un élément d'accrochage (52) qui, lors de l'extraction du dispositif, peut être saisi par l'arrière par un instrument d'extraction (53, 54, 55).

18. Dispositif selon la revendication 17, **caractérisé en ce que** l'élément d'accrochage est constitué par un fil (52) tendu transversalement sur l'ouverture distale du cathéter et qui, lors de l'introduction du dispositif, est en même temps un élément d'appui pour un dispositif d'introduction.

19. Dispositif selon l'une quelconque des revendications 14 à 18, **caractérisé en ce qu'**il comporte une tige (43) permettant son introduction dans l'urètre masculine, pouvant être fichée sur l'extrémité distale de l'élément distal (2) et dans laquelle un noyau en forme de barre (50) est guidé de manière mobile, noyau dont la section terminale avant (50b) peut être sortie de la tige (43) pour venir en contact avec l'extrémité distale du cathéter.

20. Dispositif selon la revendication 19, **caractérisé en ce que** la section terminale avant (50b) du noyau (50) peut être introduite jusqu'à une butée (51) dans le lumen du cathéter et, une fois introduite, enjambe en la raidissant un point de division du cathéter composé d'un élément proximal et d'un élément distal (1 ou 2).

21. Dispositif selon la revendication 20, **caractérisé en ce que** la section terminale avant (50b) a une section transversale réduite relativement au noyau (50) et que la butée est un épaulement (51).

22. Dispositif selon l'une quelconque des revendications 19 à 21, **caractérisé en ce que** l'extrémité arrière (50a) du noyau en forme de barre (50) dépasse de la tige (43) en faisant office d'extrémité d'actionnement.

23. Dispositif selon l'une quelconque des revendications 19 à 22, **caractérisé en ce que** le dispositif d'introduction est réalisé en même temps sous forme d'un dispositif de remplissage permettant de remplir les ballons de fluide du fait que l'extrémité avant de la tige (43) assurant l'emboîtement est équipée de canules (48, 49) insérables dans des soupapes de liaison (19, 20), situées sur la face frontale, de l'élément de cathéter (2) et qui sont reliées par des canaux d'acheminement s'étendant dans la tige (43) à des raccordements (44, 47) formés au niveau de la tige (43).

24. Dispositif selon l'une quelconque des revendications 1 à 18, **caractérisé en ce qu'**il est prévu un élément d'accrochage (52) dans le lumen du cathéter (1, 2) et qu'une barre d'extraction (53) permettant une extraction du dispositif et pouvant être introduite dans l'urètre et à l'extrémité avant de laquelle un prolongement (54) comportant au moins un ardillon (55) est réalisé, lequel
